(19) Europäisches Patentamt
European Patent Office
Office européen des brevets

(11) **EP 3 340 871 B1**

(12) **EUROPEAN PATENT SPECIFICATION**

(45) Date of publication and mention
of the grant of the patent:
**29.11.2023 Bulletin 2023/48**

(21) Application number: **16759877.0**

(22) Date of filing: **25.08.2016**

(51) International Patent Classification (IPC):
**A61B 5/00** *(2006.01)*    **A61B 5/316** *(2021.01)*
**A61B 5/318** *(2021.01)*    **A61B 5/339** *(2021.01)*

(52) Cooperative Patent Classification (CPC):
**A61B 5/7203; A61B 5/316; A61B 5/318;**
**A61B 5/339; A61B 5/7221**

(86) International application number:
**PCT/IB2016/055061**

(87) International publication number:
**WO 2017/033140 (02.03.2017 Gazette 2017/09)**

(54) **HIGH/LOW FREQUENCY SIGNAL QUALITY EVALUATIONS OF ECG LEAD SIGNALS**

BEURTEILUNG DER HOCH-/NIEDERFREQUENZSIGNALQUALITÄT VON
EKG-ELEKTRODENSIGNALEN

ÉVALUATIONS DE QUALITÉ DE SIGNAL DE HAUTE/BASSE FRÉQUENCE DE SIGNAUX DE
DÉRIVATION D'ECG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.08.2015 US 201562209394 P**

(43) Date of publication of application:
**04.07.2018 Bulletin 2018/27**

(73) Proprietor: **Koninklijke Philips N.V.**
**5656 AG Eindhoven (NL)**

(72) Inventors:
• **FIROOZABADI, Reza**
**5656 AE Eindhoven (NL)**
• **GREGG, Richard E.**
**5656 AE Eindhoven (NL)**

(74) Representative: **Philips Intellectual Property &
Standards
High Tech Campus 52
5656 AG Eindhoven (NL)**

(56) References cited:
**US-A- 5 827 196        US-A1- 2010 022 903
US-A1- 2014 249 437**

• **SATIJA UDIT ET AL: "A simple method for
detection and classification of ECG noises for
wearable ECG monitoring devices", 2015 2ND
INTERNATIONAL CONFERENCE ON SIGNAL
PROCESSING AND INTEGRATED NETWORKS
(SPIN), IEEE, 19 February 2015 (2015-02-19),
pages 164-169, XP032768817, DOI:
10.1109/SPIN.2015.7095425 [retrieved on
2015-04-24]**

**Description**

FIELD OF THE INVENTION

The present disclosure generally relates to signal quality of each electrocardiogram ("ECG") lead in an ECG recording during continuous ECG monitoring of a patient. The present disclosure more particularly relates an evaluation and visually display of high-frequency and low-frequency noise levels within electrocardiogram segments of each ECG lead in an ECG recording during continuous ECG monitoring of a patient.

BACKGROUND OF THE INVENTION

Electrocardiography is a non-invasive procedure for recording a multi-lead electrocardiogram ("ECG") (e.g., a standard 12-lead ECG or a non-standard EASI lead ECG) as a representation of an electrical cardiac activity of a patient conducted to vector cardiogram or other ECG lead system electrodes on the body surface of the patient. Electrocardiography is utilized in a wide variety of clinical settings ranging from physical examinations for regular checkups and emergencies to monitoring for the preparation, operative and recovery phases of surgical/diagnostic procedures. The purpose of the electrocardiography for these setting is a detection and evaluation of cardiac problems including, but not limited to, arrhythmias (commonly known as an irregular heartbeat), tachycardia (commonly known as a fast heartbeat), bradycardia (commonly known as a a slow heartbeat), and myocardial infarction (commonly known as a heart attack). Thus, a high-quality ECG in terms of accuracy is imperative to a reliable cardiac diagnosis and monitoring.

More particularly, various sources of noise induced on an ECG recording may mask diagnostic features of the ECG, which may result in an inaccurate interpretation of the ECG recordings and resulting diagnosis by a monitoring device and/or operator of the monitoring device. In an extreme case, a high level of noise on the ECG recording makes it impossible to interpret the ECG at all. Clearly, a low-quality ECG may cause a high number of false alarms generated by monitoring devices, particularly in critical care units (CCU) and intensive care units (ICU), which in turn may cause fatigue in medical personnel.

One beneficial method known in the art for evaluating a signal quality of an ECG recording involves a determination of an average level of all types of noise in all leads as a single noise score for long term ECG signals and further involves a display of a compressed time-scale color-bar as the signal quality indicator. This method is very useful in rapid selection of high-quality ECG signal as representative segments to review for detailed analysis of rhythm and morphology. The present disclosure is directed to complementing the known method by determining and displaying a quality of real-time multi-lead short segments shown on a monitor screen and a reliability of each plot based on the level of noise categories including high-frequency noise and flow-frequency noise.

United States Patent Application Publication Number US 2010/0022903 A1 relates to a system and method for indicating signal quality in ECG monitoring systems.

United States Patent Number 5,827,196 presents a system for providing characterizations of waveform representations of heart function.

United States Patent Application Publication Number US 2014/0249437 A1 presents a system for measuring the ST-segment level of ECGs in real-time ECG monitoring.

SUMMARY OF THE INVENTION

For purposes of the present invention, the term "high-frequency" broadly encompasses high-frequency noise of a ECG lead as known in the art including, but not limited to, muscle artifact and electrode motion artifact of ECG leads, and the term "low-frequency" broadly encompasses low-frequency noise of a ECG lead as known in the art including, but not limited to, baseline wander.

According to an aspect of the invention, there is provided a monitoring device according to claim 1.

According to another aspect of the invention, there is provided a monitoring method according to claim 14.

For purposes of the inventions of the present disclosure, terms of the art including, but not limited to, "electrocardiograph", "electrocardiogram", "ECG lead", and "electrocardiogram segmentation" are to be interpreted as understood in the art of the present disclosure and as exemplary described herein.

For purposes of the inventions of the present disclosure, the term "controller" broadly encompasses all structural configurations of an application specific main board or an application specific integrated circuit housed within or linked to a monitoring device for controlling an application of various inventive principles of the present disclosure as subsequently described herein. The structural configuration of the controller may include, but is not limited to, processor(s), computer-usable/computer readable storage medium(s), an operating system, application module(s), peripheral device controller(s), slot(s) and port(s).

Examples of the monitoring device include, but are not limited to, diagnostic ECG monitoring devices (e.g.,

PageWriter TC cardiographs, Efficia series of cardiograph), exercise ECG monitoring devices (e.g., ST80i stress testing system), ambulatory ECG devices (Holter monitor), bed-side monitoring ECG devices (e.g., IntelliVue monitors, Sure-Signs monitors, and Goldway monitors), telemetry ECG monitoring devices (e.g., IntelliVue MX40 monitor); advanced life support products (e.g., HeartStart MRx and HeartStart XL defibrillators, and Efficia DFM100 module) serves to identify a particular application module as described and claimed herein without specifying or implying any additional limitation to the term "application module".

The foregoing forms and other forms of the present disclosure as well as various features and advantages of the present disclosure will become further apparent from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

BRIEF DESCRIPTION OF THE DRAWINGS

FIGS. 1A and 1B illustrate exemplary embodiments of a monitoring device in accordance with the inventive principles of the present disclosure.

FIG. 2 illustrates a flowchart representative of an exemplary embodiment of an a monitoring method in accordance with the inventive principles of the present disclosure.

FIG. 3 illustrates an exemplary ECG segment for ECG leads in accordance with the inventive principles of the present disclosure.

FIG. 4 illustrates an exemplary activity of an electrocardiogram in accordance with the inventive principles of the present disclosure.

FIG. 5 illustrates exemplary signal quality zones in accordance with the inventive principles of the present disclosure.

FIGS. 6-9 illustrates exemplary quality indications of the ECG leads in accordance with the inventive principles of the present disclosure.

DETAILED DESCRIPTION OF THE PREFERRED EMBODIMENTS

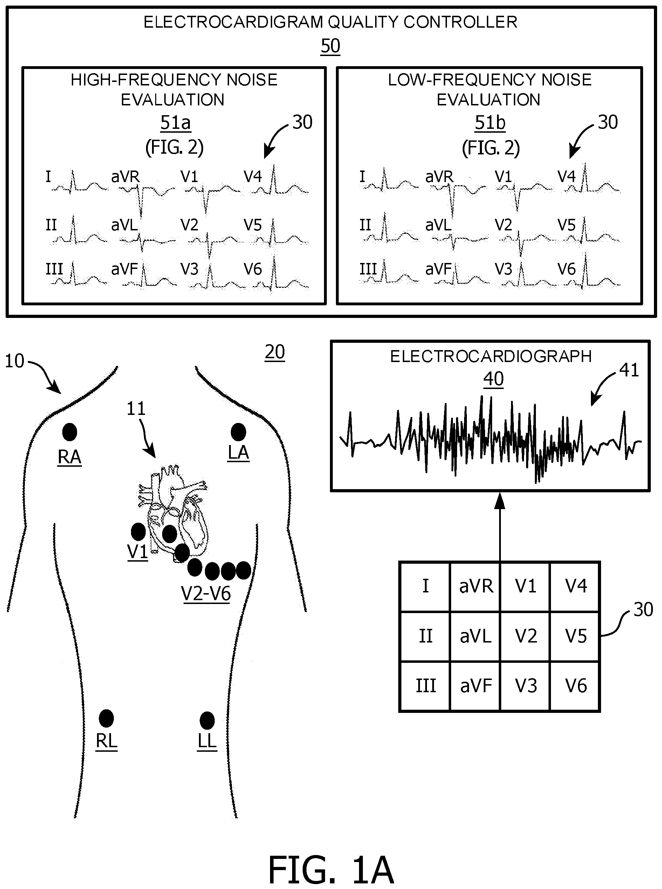

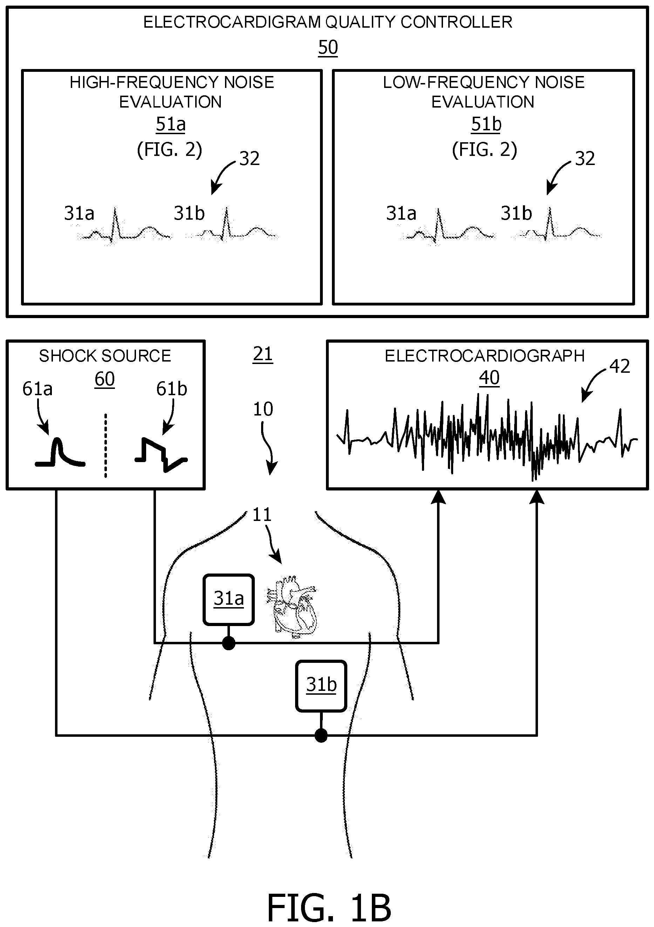

To facilitate an understanding of the present disclosure, the following description of FIGS. 1A and 1B, teaches basic inventive principles of evaluating and visually displaying a high-frequency noise level (e.g., muscle artifact and electrode motion artifact) and a low-frequency noise level (e.g., baseline wander) within electrocardiogram segments of each ECG lead in an ECG recording during a continuous ECG monitoring of a patient. From this description, those having ordinary skill in the art will appreciate how to apply the inventive principles of the present disclosure to a variety of monitoring devices displaying segments of a single lead or multi-lead continuous physiological waveform, which is often disturbed by noise or artifact that limits the interpretation and analysis of the physiological waveform, whereby the inventions of the present disclosure provides an evaluation and display of a level of noise from different sources in each individual lead.

Referring to FIG. 1A, a monitoring device 20 employs an electrocardiograph 40 and an ECG quality controller 50. In practice, ECG quality controller 50 may be segregated or integrated with electrocardiograph 40.

In operation, a strategic placement of ten (10) electrodes on a body surface of a patient 10 relative to a heart 11 of patient 10 as known in the art for establishing ECG leads 30 listed as follows:

1. Lead I: electrode RA (-) to electrode LA (+);
2. Lead II: electrode RA (-) to electrode LL (+);
3. Lead III: electrode LA (-) to electrode LL (+);
4. Lead aVR: electrode RA (+) to electrodes LA & LL (-);
5. Lead aVL: electrode LA (+) to electrodes RA & LL (-);
6. Lead aVF: electrode LL (+) to electrodes RA & LA (-);
7. Lead V1: electrode V1;
8. Lead V2: electrode V2;
9. Lead V3: electrode V3;
10. Lead V4: electrode V4;
11. Lead V5: electrode V5; and
12. Lead V6: electrode V6.

ECG leads 30 are connected to electrocardiograph 40 via cables (not shown) attached to the electrodes as known in the art to conduct electrical activity of heart 11 of patient 10 to electrocardiograph 40.

Electrocardiograph 40 is structurally configured as known in the art to process ECG leads 30 for measuring and recording an electrocardiogram 41 of heart 11 of patient 10. For embodiments of the inventions of the present disclosure incorporating a segregation of electrocardiograph 40 and ECG quality controller 50, electrocardiograph 40 may employ a digital signal processor (not shown) or a central processing unit (not shown) for processing ECG leads 30 on behalf of ECG quality controller 50.

As shown in FIG. 1A, ECG quality controller 50 executes a high frequency noise evaluation 51a and a low frequency noise evaluation 51b of processed ECG leads 30 for respectively estimating a high-frequency noise level and a low-frequency noise level of each individual ECG lead 30 on an electrocardiogram segmentation basis.

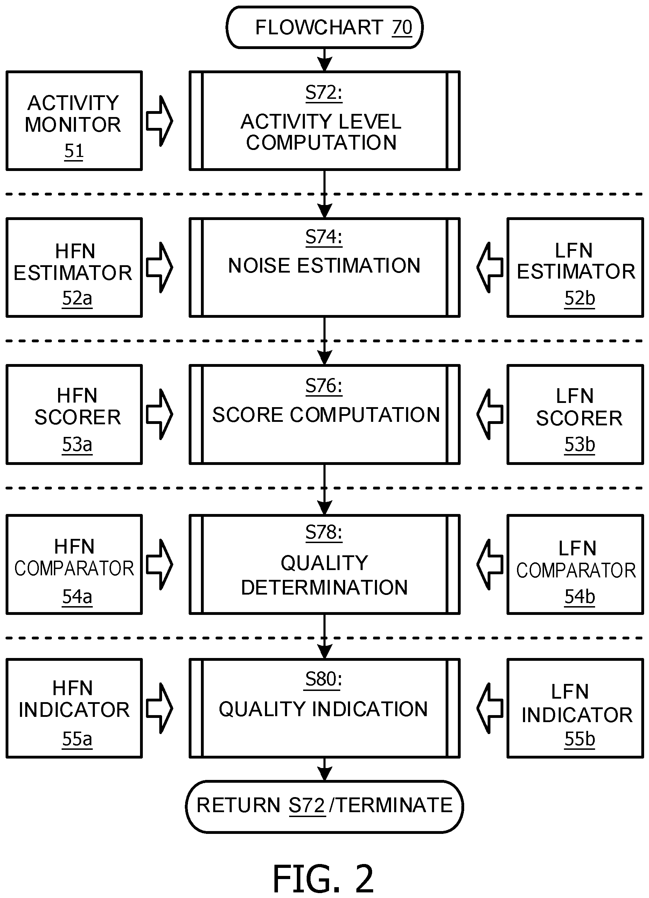

For high frequency noise evaluation 51a, ECG quality controller 50 analyzes each segment of electrocardiogram 41 in sequence to estimate a high-frequency noise level of each individual ECG lead 30 including, but not limited to, any muscle artifact and any electrode motion artifact. In practice, ECG quality controller 50 may analyze any parameter(s) of each segment of electrocardiogram 41 suitable for estimating the high-frequency noise level of each individual ECG lead 30. In one embodiment, ECG quality controller 50 analyzes a standard deviation of a short segment of electrocardiogram 41 outside of the areas of high amplitude signal from the heart for estimating the high-frequency noise level of each individual ECG lead 30 as will be further described herein and illustrated in FIG. 2. Alternative embodiments for measuring high frequency noise in a narrow (in time) window include, but are not limited to, (1) a high pass filter followed by a root mean square (RMS) calculation in a sliding window , (2) a first difference or derivative estimator followed by a RMS calculation in a sliding window, and (3) high frequency filters from a filter bank with a smoothed envelope calculation at the output of each filter.

For low frequency noise evaluation 51b, ECG quality controller 50 analyzes each segment of electrocardiogram 41 in sequence to estimate a low-frequency noise level of each individual ECG lead 30 including, but not limited to, any baseline wander of each segment of electrocardiograph 41. In practice, ECG quality controller 50 may analyze any parameter(s) of each segment of electrocardiogram 41 suitable for estimating the low-frequency noise level of each individual ECG lead 30. In one embodiment, ECG quality controller 50 analyzes each change in baseline levels of each segment of electrocardiogram 41 for estimating the low-frequency noise level of each individual ECG lead 30 as will be further described herein and illustrated in FIG. 2.

For both high/low-frequency noise evaluations 51a and 51b, ECG quality controller 50 controls a display of a signal quality indication for each segment of the electrocardiogram 41 resulting from the respective noise level analysis of each segment of electrocardiogram 41. In practice, ECG quality controller 50 may generate signal quality indication in any form suitable for communicating the results from the respective noise level analysis of each segment of electrocardiogram 41. In one embodiment, ECG quality controller 50 generates a textual signal quality indicator, a graphical signal quality indicator and/or a color-coded signal quality indicator for concurrent segment display with each ECG lead 30 as will be further described herein and illustrated in FIG. 2.

The result of high/low-frequency noise evaluations 51a and 51b is a capability of monitoring device 20 and/or an operator thereof to discard any low-quality segments of cardiogram 41 and to maintain good-quality leads among ECG leads 30 while resolving corrupted leads among ECG leads 30 as indicated by the evaluation of the high/low frequency signal quality of each individual ECG lead 30. This is beneficial to an accurate interpretation and diagnosis of electrocardiogram 41.

Referring to FIG. 1B, a defibrillator 21 employs electrocardiograph 40 and ECG quality controller 50 of monitoring device 20 as described in FIG. 1A, and further employs a shock source 60 structurally configured as known in the art to store electric energy for delivery of a defibrillation shock 61 via electrode pads 31 to heart 11 of patient 10. In practice, defibrillation shock 61 may have any waveform as known in the art. Examples of such waveforms include, but are not limited to, a monophasic sinusoidal waveform (positive sine wave) 61a and a biphasic truncated waveform 61b as shown in FIG. 1B.

In one embodiment, shock source 60 employs a high voltage capacitor bank (not shown) for storing a high voltage via a high voltage charger and a power supply upon a pressing of a charge button. Shock source 60 further employs a switching/isolation circuit (not shown) for selectively applying a specific waveform of an electric energy charge from the high voltage capacitor bank to electrode pads 31.

Electrode pads 31 are structurally configured as known in the art to be conductively applied to a patient 10 in an anterior-apex arrangement as shown in FIG. 1 or in an anterior-posterior arrangement (not shown). Electrode pads 31 conduct a defibrillation shock from shock source 60 to a heart 11 of patient 10, and are connected to electrocardiograph 40 via cables (not shown) attached to the electrodes as known in the art to conduct electrical activity of heart 11 of patient 10 to electrocardiograph 40 and for establishing ECG leads 32.

Electrocardiograph 40 is structurally configured as known in the art to process ECG leads 32 for measuring and recording an electrocardiogram 42 of heart 11 of patient 10. For embodiments of the inventions of the present disclosure incorporating a segregation of electrocardiograph 40 and ECG quality controller 50, electrocardiograph 40 may employ a digital signal processor (not shown) for streaming processed ECG leads 32 to ECG quality controller 50 or ECG quality controller 50 may employ known modules (not shown) for processing ECG leads 32. For embodiments of the inventions of the present disclosure incorporating an integration of electrocardiograph 40 and ECG quality controller 50, electrocardiograph 40 may employ a digital signal processor (not shown) or a central processing unit (not shown) for processing ECG leads 32 on behalf of ECG quality controller 50.

As shown in FIG. 1B, ECG quality controller 50 executes high frequency noise evaluation 51a and low frequency noise evaluation 51b as previously described herein of processed ECG leads 32 for respectively estimating a high-frequency noise level and a low-frequency noise level of each individual ECG lead 32 on an electrocardiogram segmentation basis. This is beneficial to an accurate interpretation and diagnosis of electrocardiogram 42.

To facilitate a further understanding of high frequency noise evaluation 51a and low frequency noise evaluation 51b in accordance with the inventive principles of the present disclosure, a flowchart 70 representative of a monitoring method of the present disclosure as executed by application modules 51-55b of an exemplary embodiment of ECG quality controller 50 as illustrated in FIG. 2 will now be described herein in the context of ECG leads 30 and electrocardiogram 40 as illustrated in FIG. 1A. From this description, those having ordinary skill in the art will appreciate how to apply the inventive principles of the present disclosure in structurally configuring various embodiments of ECG quality controller 50 in implementing various embodiments of high frequency noise evaluation 51a and low frequency noise evaluation 51b.

Referring to FIG. 2, a stage S72 of flowchart 70 encompasses an activity monitor 51 of ECG quality controller 50 computing an activity level of electrocardiogram 40. In practice, activity monitor 51 divides electrocardiogram 40 into segments of a specified duration and for each segment, computes an activity function for each sample across all ECG leads 30.

In one embodiment, activity monitor divides electrocardiogram 50 into ten (10) second ECG recording segments, which are further divided into one (1) second sections, and computes the activity function at each sample across all leads in an ECG segment is in accordance with the following equation [1]:

$$A_i = \sum_{leads}(ECG_i - ECG_{i-1})^2 \qquad [1]$$

where $A_i$ is the activity function,
where $ECG_i$ is the current ECG sample, and
wherein $ECG_{i-1}$ is the previous ECG sample.

Activity monitor 51 low-pass filters the computed values activity function $A_1$ and detects local minima of activity function $A_1$ by a short-term sliding window for each section of the ECG segment.

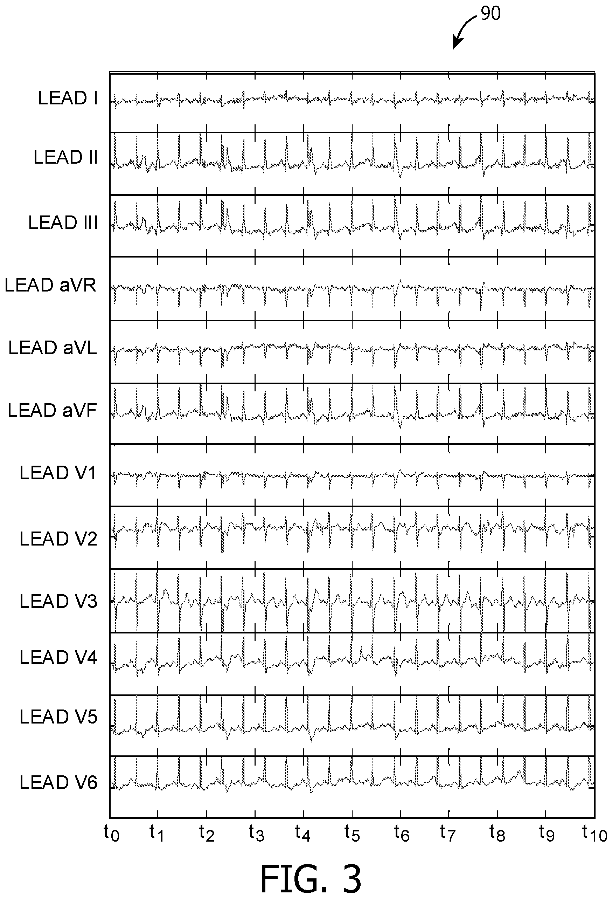

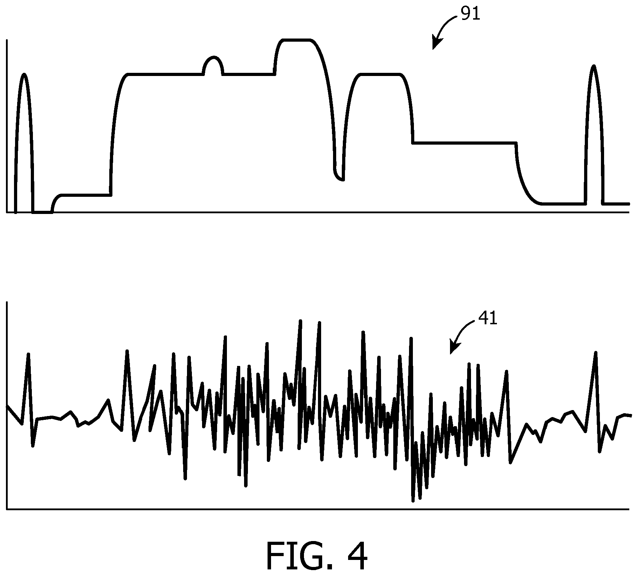

For example, FIG. 3 illustrates a ten (10) second ECG segment 90 of ECG leads 30 subdivided into ten (10) one (1)-second sections, and FIG. 4 illustrates an activity function 91 computed by activity monitor 51 within one of the sections of the ECG segment whereby activity monitor 51 detects a local minimum of activity function $A_1$ for evaluating the high-frequency noise level and the low-frequency noise level for each ECG lead 30 in a small window around the local minimum.

Referring back to FIG. 2, a stage S74 of flowchart 70 encompasses a high-frequency noise ("HFN") estimator 52a of ECG quality controller 50 for estimating a high-frequency noise level for a current ECG segment of each individual ECG lead 30. In practice, HFN estimator 52a computes a standard deviation of a short segment for each individual ECG lead 30 per segment section as an estimation of high-frequency noise level for each individual ECG lead 30. In one embodiment, HFN estimator 52a computes a standard deviation of each individual ECG lead 30 per segment section in accordance with the following equation [2]:

$$\sigma_{lead} = \sqrt{E[(ECG_{lead} - E[ECG_{lead}])^2]} \qquad [2]$$

where $\sigma_{lead}$ is the standard deviation of a particular ECG lead 30,
where $ECG_{lead}$ is the particular ECG lead 30, and
wherein $E$ is an average or expected value of standard deviation $\sigma_{lead}$ of the particular ECG lead 30.

Upon computation of standard deviation $\sigma_{lead}$ for each ECG lead 30 over each segment section, a stage S76 of flowchart 70 encompasses a HFN scorer 53a computing a HFN score for individual ECG lead 30 for the current ECG segment. In practice, HFN scorer 53a computes the HFN score of each individual ECG lead 30 per segment section as a function of the estimated high-frequency noise levels per segment section during stage S74. In one embodiment, HFN scorer 53a computes a HFN score in accordance with the following equation [3]:

$$NS_{HF} = \alpha. median(\sigma_{lead}) \qquad [3]$$

where $NS_{HF}$ is the HFN score for a particularly ECG lead 30,
where $median(\sigma_{lead})$ is the median of the computed standard deviations across sections of a time segment of a particular ECG lead 30, and
where a is a weighting factor for all ECG leads 30.

Still referring to FIG. 2, stage S74 of flowchart 70 further encompasses a low-frequency noise ("LFN") estimator 52b of ECG quality controller 50 for estimating a low-frequency noise level for a current ECG segment of each individual ECG lead 30. In practice, LFN estimator 52b computes a baseline wander per segment section as an estimation of low-frequency noise level for each individual ECG lead 30. In one embodiment, LFN estimator 52b computes baseline wander of each individual ECG lead 30 per segment section around the local minimum of activity function $A_i$ is in accordance with the following equations [4a] and [4b]:

$$BW_{1,lead} = \begin{cases} \text{Baseline}[ECG_k] - \text{Baseline}[ECG_{k-1}], & k \neq 1 \\ 0, otherwise \end{cases} \qquad [4a]$$

$$BW_{2,lead} = \begin{cases} \text{Baseline}[ECG_k] - \text{Baseline}[ECG_{k-2}], & k \neq 1,2 \\ 0, otherwise \end{cases} \qquad [4b]$$

where $BW_{1,lead}$ is the baseline wander of a current segment for a particular ECG lead 30,
where $BW_{2,lead}$ is the baseline wander of the previous segment for a particular ECG lead 30,
where $ECG_k$ is the current section of the current ECG segment (e.g., current 1-second section of the current 10-sec ECG segment), and
wherein $ECG_{k-1}$ is previous section of the current ECG segment (e.g., previous 1-second section of the current 10-sec ECG segment).

Note equations [4a] and [4b] facilitate a location of a time point which does not include high amplitude physiological signal from the heart, but rather a quiet time for the heart, in absence of physiological signal.
Upon computation of the baseline wander for each ECG lead 30 over segments, stage S76 of flowchart 70 further encompasses a LFN scorer 53b computing a LFN score for each individual ECG lead 30 for the current ECG segment. In practice, LFN scorer 53b computes the LFN score of each individual ECG lead 30 per segment section as a function of the baseline wander computed during stage S74. In one embodiment, LFN scorer 53b computes a LFN score in accordance with the following equation [5]:

$$NS_{BW} = \beta. \left( \left| BW_{1,lead} \right| + \left| BW_{2,lead} \right| \right) \qquad [5]$$

where $NS_{BW}$ is the LFN score for a particularly ECG lead 30; and
where $\beta$ is a weighting factor for all ECG leads 30.

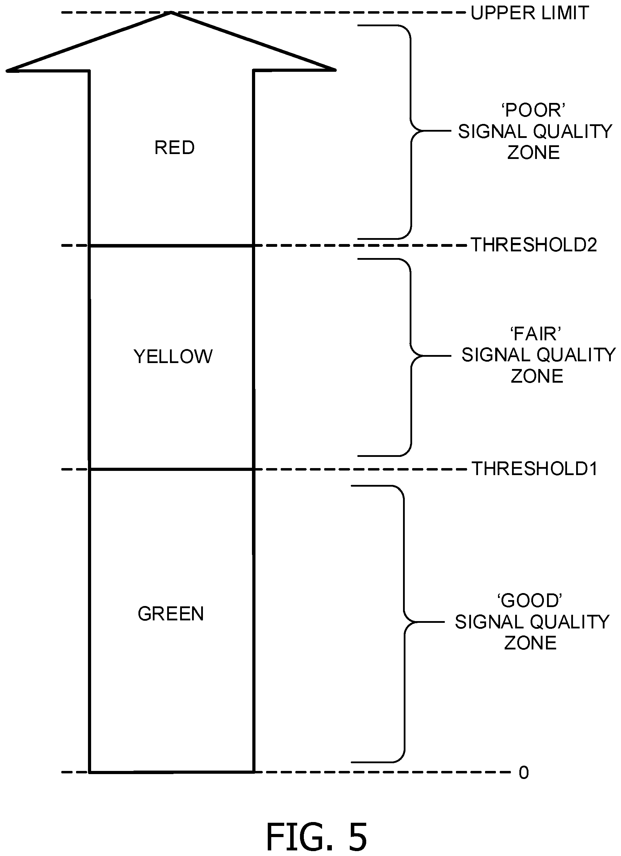

Still referring to FIG. 2, a stage S78 of flowchart 70 encompasses a HFN comparator 54a of ECG quality controller 50 comparing the HFN score of each individual ECG lead 30 to one or more signal quality threshold differentiating two or more signal quality zones, and a LFN comparator 54b of ECG quality controller 50 comparing the LFE score of each individual ECG lead 30 to the signal quality threshold(s) differentiating the signal quality zones. In practice, the signal quality threshold(s) may be selected in a validation process by training an algorithm with an annotated database and optimizing the performance of noise quality classification for classifying the HFN score in multiple signal quality zones. In one embodiment, two (2) thresholds were utilized to classify the HFN score of each individual ECG lead 30 into one of three (3) categories including (1) good, (2) fair, and (3) poor quality.
For example, the signal quality zones are summarized in the following TABLE 1 and illustrated in FIG. 5:

TABLE 1

| Signal quality zones | Criteria |
|---|---|
| Good | $0 \leq$ Noise Score $\leq$ Threshold1 |
| Fair | Threshold1 < Noise Score $\leq$ Threshold2 |
| Poor | Threshold2 < Noise Score $\leq$ UpperLimit |

Referring back to FIG. 2, upon determining the HFN and LFN signal quality of each ECG lead for the current ECG segment, a stage S80 of flowchart 70 encompasses a HFN indicator 55a and a LFN indicator 55b generating a signal quality indication representative of the signal quality. In practice, the signal quality indication may take any form suitable for communicating the HFN and LFN signal quality for each ECG lead including a textual signal quality indication and/or a graphical signal quality indication, both of which may be coded by various features including color and font. Additionally in practice, the signal quality indication may be displayed with the current ECG segment of ECG leads 30 or the representative beat of the current ECG segment.

In one embodiment, HFN indicator 55a and LFN indicator 55b normalize the respective HFN score and the LFN score to integers on a scale of 0 to 10 where Threshold1 and Threshold2 are located at 2.5 and 4.5, respectively, and the UpperLimit is 10 as shown in in accordance with the following TABLE 2:

TABLE 2

| Signal quality zones | Criteria |
|---|---|
| Good | Noise Score = 0, 1, 2 |
| Fair | Noise Score = 3,4 |
| Poor | Noise Score = 5, 6, 7, 8, 9, 10 |

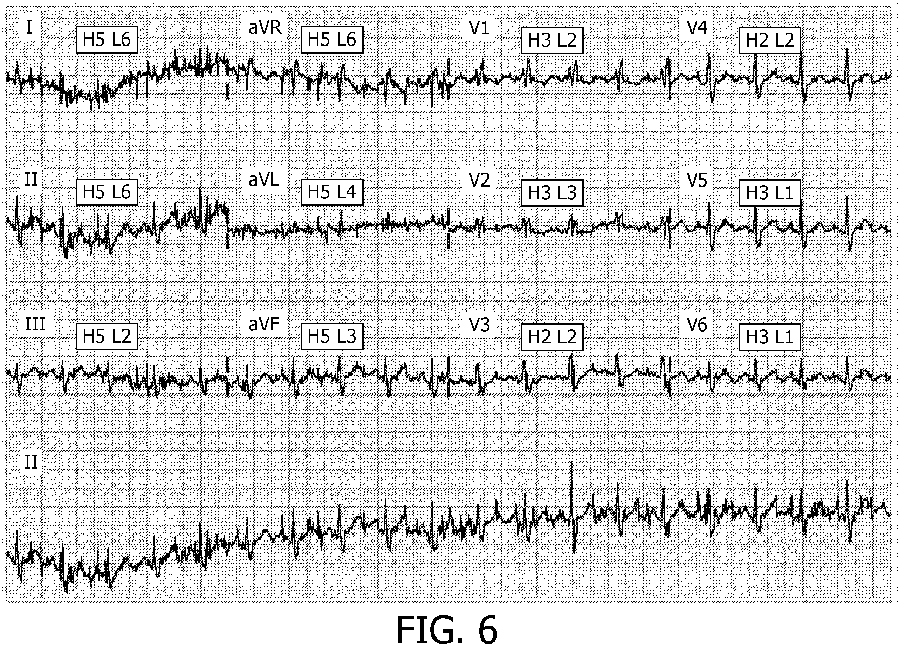

From the normalization, a color-coded textual indicator for each ECG lead may be concurrently displayed with the ECG leads 30 as exemplary shown in FIGS. 6, 7 and 9. Alternatively, the ECG lead waveforms may be color-coded as exemplary shown in FIG. 10.

Referring to FIG. 6, the following exemplary HFN scores and LFN scores are exemplary displayed adjacent an exemplary current ECG segment of ECG leads 30:

　　1. Good HFN/LFN Scores (green color-coded): Leads V3 and V4;
　　2. Fair HFN/LFN Scores (yellow color-coded): Leads aVL, V1, V2, V5 and V6; and
　　3. Poor HFN/LFN Scores (red color-coded): Leads I, II, III, aVR and aVF.

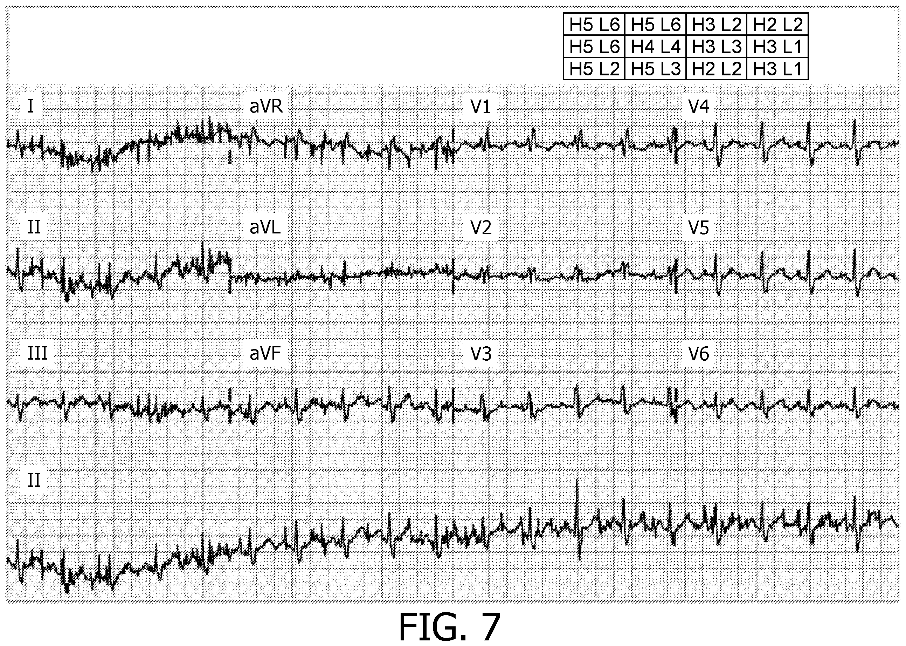

Referring to FIG. 7, the following exemplary HFN scores and LFN scores are exemplary displayed in boxes above an exemplary current ECG segment of ECG leads 30:

　　1. Good HFN/LFN Scores (green color-coded): Leads V3 and V4;
　　2. Fair HFN/LFN Scores (yellow color-coded): Leads aVL, V1, V2, V5 and V6; and
　　3. Poor HFN/LFN Scores (red color-coded): Leads I, II, III, aVR and aVF.

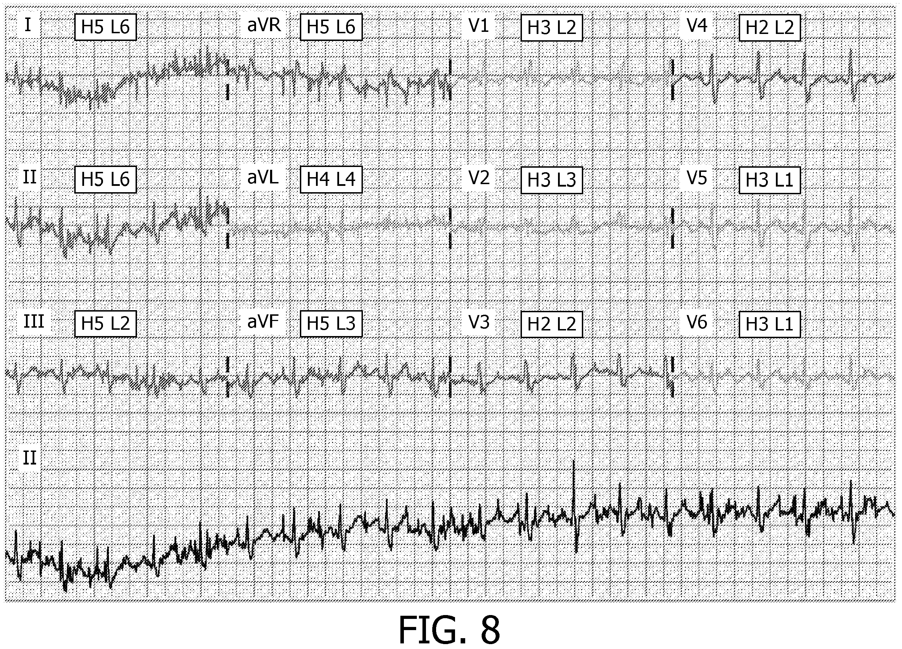

Referring to FIG. 8, the following exemplary HFN scores and LFN scores are exemplary displayed adjacent an exemplary color-coded current ECG segment of ECG leads 30:

　　1. Good HFN/LFN Scores (green wave): Leads V3 and V4;
　　2. Fair HFN/LFN Scores (yellow wave): Leads aVL, V1, V2, V5 and V6; and
　　3. Poor HFN/LFN Scores (red wave): Leads I, II, III, aVR and aVF.

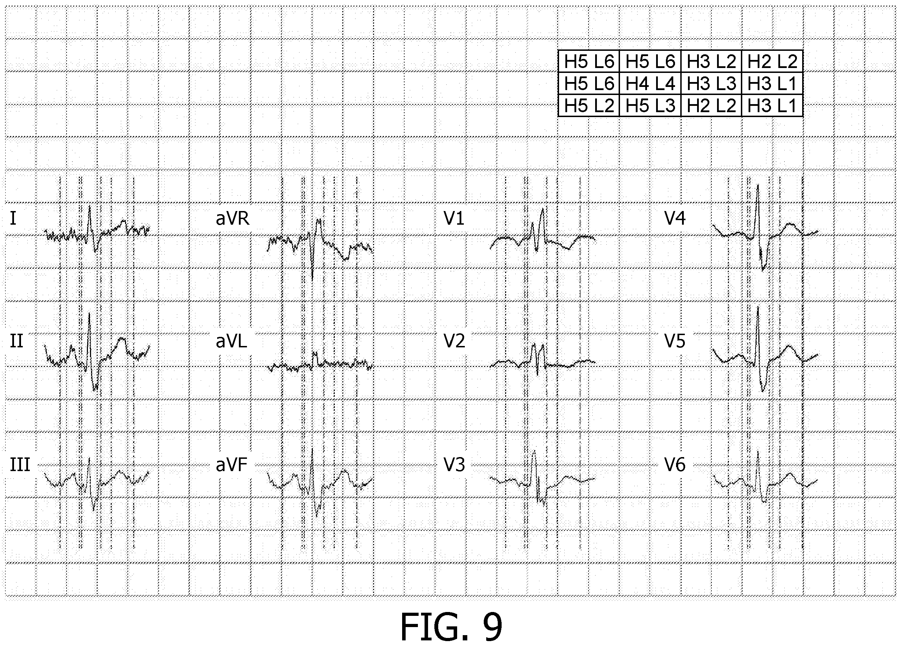

Referring to FIG. 9, the following exemplary HFN scores and LFN scores are exemplary displayed in boxes above an exemplary representative beats of the current ECG segment of ECG leads 30:

　　1. Good HFN/LFN Scores (green color-coded): Leads V3 and V4;
　　2. Fair HFN/LFN Scores (yellow color-coded): Leads aVL, V1, V2, V5 and V6; and

3. Poor HFN/LFN Scores (red color-coded): Leads I, II, III, aVR and aVF.

Referring back to FIG. 2, note one of the purposes of stage S76 as described herein is to equalize the HFN score and the LFN score for comparisons to the same signal quality thresholds of stage S78. Nonetheless, in practice, the HFN score and the LFN score do not have to be equalized and may be compared to different signal quality thresholds.

Upon completion of stage S80, flowchart 70 returns to stage S72 to repeat stages S72-S80 for the next ECG segment and continues to loop until terminated.

Referring to FIGS. 1-9, those having ordinary skill in the art will appreciate numerous benefits of the present disclosure including, but not limited to, the novel and unique evaluating and visually displaying high-frequency and low-frequency noise levels within electrocardiogram segments of each ECG lead in an ECG recording during a continuous ECG monitoring of a patient. More generally, the inventions of the present disclosure applies to a variety of monitoring devices for displaying segments of a single lead or multi-lead continuous physiological waveform, which are often disturbed by noise or artifact that limits the interpretation and analysis of the physiological waveform, whereby the inventions of the present disclosure provides an evaluation and display of a level of noise from different sources in each individual lead. The benefits being a capability of the monitoring device and/or an operator thereof to discard any low-quality segments of the physiological waveform and to maintain good-quality leads while resolving corrupted leads as indicated by the evaluation of the high/low frequency signal quality of each individual lead.

Furthermore, as one having ordinary skill in the art will appreciate in view of the teachings provided herein, features, elements, components, etc. described in the present disclosure/specification and/or depicted in the FIGS. 1-9 may be implemented in various combinations of electronic components/circuitry, hardware, executable software and executable firmware and provide functions which may be combined in a single element or multiple elements. For example, the functions of the various features, elements, components, etc. shown/illustrated/depicted in the FIGS. 1-9 can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" should not be construed to refer exclusively to hardware capable of executing software, and can implicitly include, without limitation, digital signal processor ("DSP") hardware or other central processing unit ("CPU"),, memory (e.g., read only memory ("ROM") for storing software, random access memory ("RAM"), non-volatile storage, etc.) and virtually any means and/or machine (including hardware, software, firmware, circuitry, combinations thereof, etc.) which is capable of (and/or configurable) to perform and/or control a process.

It will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Furthermore, exemplary embodiments of the present disclosure can take the form of a computer program product or application module (by itself not falling under the scope of the claim) accessible from a computer-usable and/or computer-readable storage medium providing program code and/or instructions for use by or in connection with, e.g., a computer or any instruction execution system. In accordance with the present disclosure, a computer-usable or computer readable storage medium can be any apparatus that can, e.g., include, store, communicate, propagate or transport the program for use by or in connection with the instruction execution system, apparatus or device. Such exemplary medium can be, e.g., an electronic, magnetic, optical, electromagnetic, infrared or semiconductor system (or apparatus or device) or a propagation medium. Examples of a computer-readable medium include, e.g., a semiconductor or solid state memory, magnetic tape, a removable computer diskette, a random access memory (RAM), a read-only memory (ROM), flash (drive), a rigid magnetic disk and an optical disk. Current examples of optical disks include compact disk - read only memory (CD-ROM), compact disk - read/write (CD-R/W) and DVD. Further, it should be understood that any new computer-readable medium which may hereafter be developed should also be considered as computer-readable medium as may be used or referred to in accordance with exemplary embodiments of the present disclosure.

Having described preferred and exemplary embodiments of high/low-frequency noise evaluations of an electrical connection to an electrocardiograph, it is noted that modifications and variations can be made by persons having ordinary skill in the art in light of the teachings provided herein, including the FIGS. 1-9.

Moreover, it is contemplated that corresponding and/or related systems incorporating and/or implementing the device or such as may be used/implemented in a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure, but do not fall within the scope of the claims.

**Claims**

1. A monitoring device, comprising:

   a monitor screen,
   an electrocardiograph (40) adapted to derive an electrocardiogram from at least one of ECG lead (30) responsive to a connection of the electrocardiograph (40) to the at least one ECG lead (30);
   an ECG quality controller (50) adapted, in response to the connection of the electrocardiograph (40) to the at least one ECG lead (30),

   - to control separate evaluations of a high-frequency noise level and a low-frequency noise level of an individual ECG lead (30) on an electrocardiogram segmentation basis, and
   - to ascertain, in the high-frequency noise level evaluation of the individual ECG lead (30), the standard deviation of an electrocardiogram segment for each individual ECG lead (30) per segment section as an estimation of the high-frequency noise level for each individual ECG lead (30), the electrocardiogram segment being divided into a plurality of segment sections;

   wherein the ECG quality controller (50) is further adapted to control the monitor screen to display the separate evaluations of the high-frequency noise level and the low-frequency noise level of the individual ECG lead (30) on an electrocardiogram segmentation basis.

2. The monitoring device of claim 1, wherein the high-frequency noise level evaluation of the individual ECG lead (30) by the ECG quality controller (50) includes:
   the ECG quality controller (50) being adapted, during each electrocardiogram segment, to derive a high-frequency noise score for the individual ECG lead (30) from a standard deviation of the individual ECG lead (30).

3. The monitoring device of claim 1, wherein the high-frequency noise level evaluation of the individual ECG lead (30) by the ECG quality controller (50) further includes:
   the ECG quality controller (50) being adapted, during each electrocardiogram segment, to scale the standard deviation of the individual ECG lead (30) by a weighting factor to thereby derive the high-frequency noise score for the individual ECG lead (30).

4. The monitoring device of claim 1, wherein the high-frequency noise level evaluation of the individual ECG lead (30) by the ECG quality controller (50) further includes:
   the ECG quality controller (50) being adapted, during each electrocardiogram segment, to compare the high-frequency noise score to at least one signal quality threshold differentiating a plurality of distinct signal quality zones.

5. The monitoring device of claim 1, wherein the low-frequency noise level evaluation of the individual ECG lead (30) by the ECG quality controller (50) includes:
   the ECG quality controller (50) being adapted, during each electrocardiogram segment, to derive a low-frequency noise score for the individual ECG lead (30) from any change in a baseline level of the individual ECG lead (30).

6. The monitoring device of claim 5, wherein the low-frequency noise level evaluation of the individual ECG lead (30) by the ECG quality controller (50) further includes:
   the ECG quality controller (50) being adapted, during each electrocardiogram segment, to scale any change in the baseline level of the individual ECG lead (30) by a weighting factor to thereby derive the low-frequency noise score of the individual ECG lead (30).

7. The monitoring device of claim 5, wherein the low-frequency noise level evaluation of the individual ECG lead (30) by the ECG quality controller (50) further includes:
   the ECG quality controller (50) being adapted to ascertain any change in the baseline level of the individual ECG lead (30) measured at a specified activity level of each electrocardiogram segment.

8. The monitoring device of claim 5, wherein the low-frequency noise level evaluation of the individual ECG lead (30) by the ECG quality controller (50) further includes:
   the ECG quality controller (50) being adapted to compare the low-frequency noise score to at least one signal quality threshold differentiating a plurality of distinct signal quality zones.

9. The monitoring device of claim 1, wherein the display of the high-frequency noise level evaluation and the low-frequency noise level evaluation of the individual ECG lead (30) by the ECG quality controller (50) includes:
the ECG quality controller (50) being adapted to display one of the individual ECG lead (30) or a representative beat of the ECG lead (30) concurrently with a signal quality indication of the separate evaluations of the high-frequency noise level and the low-frequency noise level of the individual ECG lead (30),
wherein each signal quality indication is at least one of a textual signal quality indication and a graphical signal quality indication.

10. The monitoring device of claim 1, wherein the ECG quality controller (50) includes:

a high-frequency noise estimator adapted to compute a median of standard deviations of the individual ECG lead (30) measured at specified activity levels during an electrocardiogram segment; and
a high-frequency noise scorer adapted to derive a high-frequency noise score from a computation by the high-frequency noise estimator of the median of standard deviations for the individual ECG lead (30); and
a high-frequency noise comparator adapted to compare a computation by the high-frequency noise scorer of the high-frequency noise score to at least one signal quality threshold differentiating a plurality of distinct signal quality zones.

11. The monitoring device of claim 10, wherein the ECG quality controller (50) further includes:
a high-frequency noise indicator adapted to derive a signal quality indication of the evaluation of the high-frequency noise level of the individual ECG lead (30) from a comparison by the high-frequency noise comparator of the high-frequency noise score to at least one signal quality threshold,
wherein each signal quality indication is at least one of a textual signal quality indication and a graphical signal quality indication.

12. The monitoring device of claim 1, wherein the ECG quality controller (50) includes:

a low-frequency noise estimator adapted to compute any change in a baseline level of the individual ECG lead (30) at specified activity level within an electrocardiogram segment; and
a low-frequency noise scorer adapted to derive a low-frequency noise score from a computation by the low-frequency noise estimator of any change in the baseline level of the individual ECG lead (30); and
a low-frequency noise comparator adapted to compare a computation by the low-frequency noise scorer of the low-frequency noise score to at least one signal quality threshold differentiating a plurality of distinct signal quality zones.

13. The monitoring device of claim 12, wherein the ECG quality controller (50) further includes:
a low-frequency noise indicator adapted to derive a signal quality indication of the evaluation of the low-frequency noise level of the individual ECG lead (30) from a comparison by the low-frequency noise comparator of the low-frequency noise score to at least one signal quality threshold,
wherein each signal quality indication is at least one of a textual signal quality indication and a graphical signal quality indication.

14. A monitoring method, comprising

deriving, by an electrocardiograph (40), an electrocardiogram from at least one ECG lead (30) responsive to a connection of the electrocardiograph (40) to the at least one ECG lead (30);
controlling, by an ECG quality controller (50), separate evaluations of a high-frequency noise level and a low-frequency noise level of an individual ECG lead (30) on an electrocardiogram segmentation basis;
ascertaining, in the high-frequency noise level evaluation of the individual ECG lead (30) by the ECG quality controller (50), the standard deviation of an electrocardiogram segment for each individual ECG lead (30) per segment section as an estimation of the high-frequency noise level for each individual ECG lead (30), the electrocardiogram segment being divided into a plurality of segment sections; and
displaying, on a monitor screen, the separate evaluations of the high-frequency noise level and the low-frequency noise level of the individual ECG lead (30) on an electrocardiogram segmentation basis.

**Patentansprüche**

1. Überwachungsvorrichtung, umfassend:

   ein Monitorbildschirm,
   einen Elektrokardiographen (40), der ausgelegt ist, um als Reaktion auf eine Verbindung des Elektrokardiographen (40) mit der mindestens einen EKG-Ableitung (30) ein Elektrokardiogramm von mindestens einer EKG-Ableitung (30) abzuleiten;
   eine EKG-Qualitätssteuerung (50), die als Reaktion auf die Verbindung des Elektrokardiographen (40) mit der mindestens einen EKG-Ableitung (30) angepasst ist,

   - zum Steuern getrennter Auswertungen eines Hochfrequenzgeräuschpegels und eines Niederfrequenzgeräuschpegels einer einzelnen EKG-Ableitung (30) auf der Basis einer Elektrokardiogramm-Segmentierung, und
   - zur Ermittlung der Standardabweichung eines Elektrokardiogrammsegments für jede einzelne EKG-Ableitung (30) pro Segmentabschnitt bei der Auswertung des Hochfrequenzgeräuschpegels der einzelnen EKG-Ableitung (30) als Schätzung des Hochfrequenzgeräuschpegels für jede einzelne EKG-Ableitung (30), wobei das Elektrokardiogrammsegment in mehrere Segmentabschnitte unterteilt ist;

   wobei die EKG-Qualitätssteuerung (50) ferner so ausgelegt ist, dass sie den Monitorbildschirm steuert, um die getrennten Auswertungen des Hochfrequenzgeräuschpegels und des Niederfrequenzgeräuschpegels der einzelnen EKG-Ableitung (30) auf der Basis einer Elektrokardiogramm-Segmentierung anzuzeigen.

2. Überwachungsvorrichtung nach Anspruch 1, wobei die Auswertung des Hochfrequenzgeräuschpegels der einzelnen EKG-Ableitung (30) durch die EKG-Qualitätssteuerung (50) umfasst:
   die EKG-Qualitätssteuerung (50), die ausgelegt ist, um während jedes Elektrokardiogrammsegments aus einer Standardabweichung der einzelnen EKG-Ableitung (30) eine Hochfrequenzgeräuschbewertung für die einzelne EKG-Ableitung (30) abzuleiten.

3. Überwachungsvorrichtung nach Anspruch 1, wobei die Auswertung des Hochfrequenzgeräuschpegels der einzelnen EKG-Ableitung (30) durch die EKG-Qualitätssteuerung (50) ferner umfasst:
   die EKG-Qualitätssteuerung (50), die ausgelegt ist, um während jedes Elektrokardiogrammsegments die Standardabweichung der einzelnen EKG-Ableitung (30) mit einem Gewichtungsfaktor zu skalieren, um dadurch die Hochfrequenzgeräuschbewertung für die einzelne EKG-Ableitung (30) abzuleiten.

4. Überwachungsvorrichtung nach Anspruch 1, wobei die Auswertung des Hochfrequenzgeräuschpegels der einzelnen EKG-Ableitung (30) durch die EKG-Qualitätssteuerung (50) ferner umfasst:
   die EKG-Qualitätssteuerung (50), die ausgelegt ist, um während jedes Elektrokardiogrammsegments die Hochfrequenzgeräuschbewertung mit mindestens einem Signalqualitätsschwellenwert zu vergleichen, der mehrere unterschiedliche Signalqualitätszonen unterscheidet.

5. Überwachungsvorrichtung nach Anspruch 1, wobei die Auswertung des Niederfrequenzgeräuschpegels der einzelnen EKG-Ableitung (30) durch die EKG-Qualitätssteuerung (50) umfasst:
   die EKG-Qualitätssteuerung (50), die ausgelegt ist, um während jedes Elektrokardiogrammsegments aus jeder Änderung eines Grundlinienpegels der einzelnen EKG-Ableitung (30) eine Niederfrequenzgeräuschbewertung für die einzelne EKG-Ableitung (30) abzuleiten.

6. Überwachungsvorrichtung nach Anspruch 5, wobei die Auswertung des Niederfrequenzgeräuschpegels der einzelnen EKG-Ableitung (30) durch die EKG-Qualitätssteuerung (50) ferner umfasst:
   die EKG-Qualitätssteuerung (50), die ausgelegt ist, um während jedes Elektrokardiogrammsegments jede Änderung des Grundlinienpegels der einzelnen EKG-Ableitung (30) mit einem Gewichtungsfaktor zu skalieren, um dadurch die Niederfrequenzgeräuschbewertung der einzelnen EKG-Ableitung (30) abzuleiten.

7. Überwachungsvorrichtung nach Anspruch 5, wobei die Auswertung des Niederfrequenzgeräuschpegels der einzelnen EKG-Ableitung (30) durch die EKG-Qualitätssteuerung (50) ferner umfasst:
   die EKG-Qualitätssteuerung (50), die ausgelegt ist, um jede Änderung des Grundlinienpegels der einzelnen EKG-Ableitung (30) zu ermitteln, gemessen bei einem bestimmten Aktivitätsniveau jedes Elektrokardiogrammsegments.

8. Überwachungsvorrichtung nach Anspruch 5, wobei die Auswertung des Niederfrequenzgeräuschpegels der einzelnen EKG-Ableitung (30) durch die EKG-Qualitätssteuerung (50) ferner umfasst:
die EKG-Qualitätssteuerung (50), die ausgelegt ist, um die Niederfrequenzgeräuschbewertung mit mindestens einem Signalqualitätsschwellenwert zu vergleichen, um mehrere unterschiedliche Signalqualitätszonen zu unterscheiden.

9. Überwachungsvorrichtung nach Anspruch 1, wobei die Anzeige der Auswertung des Hochfrequenzgeräuschpegels und der Auswertung des Niederfrequenzgeräuschpegels der einzelnen EKG-Ableitung (30) durch die EKG-Qualitätssteuerung (50) umfasst:

die EKG-Qualitätssteuerung (50), die ausgelegt ist, um eine der einzelnen EKG-Ableitungen (30) oder einen repräsentativen Schlag der EKG-Ableitung (30) gleichzeitig mit einer Signalqualitätsangabe der getrennten Auswertungen des Hochfrequenzgeräuschpegels und des Niederfrequenzgeräuschpegels der einzelnen EKG-Ableitung (30) anzuzeigen,
wobei jede Signalqualitätsangabe mindestens eine textuelle Signalqualitätsangabe und eine grafische Signalqualitätsangabe ist.

10. Überwachungsvorrichtung nach Anspruch 1, wobei die EKG-Qualitätssteuerung (50) umfasst:

einen Hochfrequenzgeräuschschätzer, der ausgelegt ist, um einen Median der Standardabweichungen der einzelnen EKG-Ableitung (30) zu berechnen, die bei bestimmten Aktivitätsniveaus während eines Elektrokardiogrammsegments gemessen werden; und
ein Hochfrequenzgeräusch-Bewertungsgerät, das ausgelegt ist, um aus einer Berechnung des Medianwerts der Standardabweichungen für die einzelne EKG-Ableitung (30) durch den Hochfrequenzgeräuschschätzer eine Hochfrequenzgeräuschbewertung abzuleiten; und
einen Hochfrequenzgeräuschvergleicher, der ausgelegt ist, um eine Berechnung der Hochfrequenzgeräuschbewertung durch das Hochfrequenzgeräusch-Bewertungsgerät mit mindestens einem Signalqualitätsschwellenwert zu vergleichen, der mehrere unterschiedliche Signalqualitätszonen unterscheidet.

11. Überwachungsvorrichtung nach Anspruch 10, wobei die EKG-Qualitätssteuerung (50) ferner umfasst:

einen Hochfrequenzgeräuschindikator, der ausgelegt ist, um aus einem Vergleich der Hochfrequenzgeräuschbewertung durch den Hochfrequenzgeräuschvergleicher mit mindestens einem Signalqualitätsschwellenwert eine Signalqualitätsangabe für die Auswertung des Hochfrequenzrauschpegels der einzelnen EKG-Ableitung (30) abzuleiten,
wobei jede Signalqualitätsangabe mindestens eine textuelle Signalqualitätsangabe und eine grafische Signalqualitätsangabe ist.

12. Überwachungsvorrichtung nach Anspruch 1, wobei die EKG-Qualitätssteuerung (50) umfasst:

einen Niederfrequenzgeräuschschätzer, der ausgelegt ist, um jede Änderung eines Grundlinienpegels der einzelnen EKG-Ableitung (30) bei einem bestimmten Aktivitätsniveau innerhalb eines Elektrokardiogrammsegments zu berechnen; und
ein Niederfrequenzgeräusch-Bewertungsgerät, das ausgelegt ist, um aus einer Berechnung jeglicher Änderung des Grundlinienpegels der einzelnen EKG-Ableitung (30) durch den Niederfrequenzgeräuschschätzer eine Niederfrequenzgeräuschbewertung abzuleiten; und einen Niederfrequenzgeräuschvergleicher, der ausgelegt ist, um eine Berechnung der Niederfrequenzgeräuschbewertung durch das Niederfrequenzgeräusch-Bewertungsgerät mit mindestens einem Signalqualitätsschwellenwert zu vergleichen, der mehrere unterschiedliche Signalqualitätszonen unterscheidet.

13. Überwachungsvorrichtung nach Anspruch 12, wobei die EKG-Qualitätssteuerung (50) ferner umfasst:

einen Niederfrequenzgeräuschindikator, der ausgelegt ist, um aus einem Vergleich der Niederfrequenzgeräuschbewertung durch den Niederfrequenzgeräuschvergleicher mit mindestens einem Signalqualitätsschwellenwert, eine Signalqualitätsangabe für die Auswertung des Niederfrequenzgeräuschpegels der einzelnen EKG-Ableitung (30) abzuleiten,
wobei jede Signalqualitätsangabe mindestens eine textuelle Signalqualitätsangabe und eine grafische Signalqualitätsangabe ist.

**14.** Überwachungsverfahren, umfassend

Ableiten eines Elektrokardiogramms von mindestens einer EKG-Ableitung (30) durch einen Elektrokardiographen (40) als Reaktion auf eine Verbindung des Elektrokardiographen (40) mit der mindestens einen EKG-Ableitung (30);

Steuern, durch einen EKG-Qualitätssteuerung (50), getrennter Auswertungen eines Hochfrequenzgeräuschpegels und eines Niederfrequenzgeräuschpegels einer einzelnen EKG-Ableitung (30) auf der Basis einer Elektrokardiogramm-Segmentierung;

Ermitteln der Standardabweichung eines Elektrokardiogrammsegments für jede einzelne EKG-Ableitung (30) pro Segmentabschnitt bei der Auswertung des Hochfrequenzgeräuschpegels der einzelnen EKG-Ableitungen (30) durch die EKG-Qualitätssteuerung (50) als Schätzung des Hochfrequenzgeräuschpegels für jede einzelne EKG-Ableitung (30), wobei das Elektrokardiogrammsegment in mehrere Segmentabschnitte unterteilt ist; und

Anzeigen der getrennten Auswertungen des Hochfrequenzgeräuschpegels und des Niederfrequenzgeräuschpegels der einzelnen EKG-Ableitung (30) auf der Basis einer Elektrokardiogramm-Segmentierung auf einem Monitorbildschirm.

**Revendications**

**1.** Dispositif de surveillance, comprenant:

un écran de moniteur,

un électrocardiographe (40) adapté pour dériver un électrocardiogramme à partir d'au moins une dérivation d'ECG (30) en réponse à une connexion de l'électrocardiographe (40) à l'au moins une dérivation d'ECG (30);

un dispositif de contrôle de qualité d'ECG (50) adapté, en réponse à la connexion de l'électrocardiographe (40) à l'au moins une dérivation d'ECG (30),

- pour contrôler des évaluations séparées d'un niveau de bruit à haute fréquence et d'un niveau de bruit à basse fréquence d'une dérivation d'ECG individuelle (30) sur la base d'une segmentation d'électrocardiogramme, et

- pour déterminer, lors de l'évaluation du niveau de bruit à haute fréquence de la dérivation d'ECG individuelle (30), l'écart type d'un segment d'électrocardiogramme pour chaque dérivation d'ECG individuelle (30) par section de segment en tant qu'estimation du niveau de bruit à haute fréquence pour chaque dérivation d'ECG individuelle (30), le segment d'électrocardiogramme étant divisé en une pluralité de sections de segment;

où le dispositif de contrôle de qualité d'ECG (50) est en outre adapté pour commander l'écran du moniteur afin d'afficher les évaluations séparées du niveau de bruit à haute fréquence et du niveau de bruit à basse fréquence de la dérivation d'ECG individuelle (30) sur la base d'une segmentation d'électrocardiogramme.

**2.** Dispositif de surveillance selon la revendication 1, dans lequel l'évaluation du niveau de bruit à haute fréquence de la dérivation d'ECG individuelle (30) par le dispositif de contrôle de qualité d'ECG (50) comprend:
le dispositif de contrôle de qualité d'ECG (50) qui est adapté, pendant chaque segment d'électrocardiogramme, pour dériver un score de bruit à haute fréquence pour la dérivation d'ECG individuelle (30) à partir d'un écart type de la dérivation d'ECG individuelle (30).

**3.** Dispositif de surveillance selon la revendication 1, dans lequel l'évaluation du niveau de bruit à haute fréquence de la dérivation d'ECG individuelle (30) par le dispositif de contrôle de qualité d'ECG (50) comprend en outre:
le dispositif de contrôle de qualité d'ECG (50) qui est adapté, pendant chaque segment d'électrocardiogramme, pour mettre à l'échelle l'écart type de la dérivation d'ECG individuelle (30) par un facteur de pondération afin de dériver ainsi le score de bruit à haute fréquence pour la dérivation d'ECG individuelle (30).

**4.** Dispositif de surveillance selon la revendication 1, dans lequel l'évaluation du niveau de bruit à haute fréquence de la dérivation d'ECG individuelle (30) par le dispositif de contrôle de qualité d'ECG (50) comprend en outre:
le dispositif de contrôle de qualité d'ECG (50) qui est adapté, pendant chaque segment d'électrocardiogramme, à comparer le score de bruit à haute fréquence avec au moins un seuil de qualité de signal différenciant une pluralité de zones de qualité de signal distinctes.

5. Dispositif de surveillance selon la revendication 1, dans lequel l'évaluation du niveau de bruit à basse fréquence de la dérivation d'ECG individuelle (30) par le dispositif de contrôle de qualité d'ECG (50) comprend:
le dispositif de contrôle de qualité d'ECG (50) qui est adapté, pendant chaque segment d'électrocardiogramme, pour dériver un score de bruit à basse fréquence pour la dérivation d'ECG individuelle (30) à partir de tout changement dans un niveau de base de la dérivation d'ECG individuelle (30) .

6. Dispositif de surveillance selon la revendication 5, dans lequel l'évaluation du niveau de bruit à basse fréquence de la dérivation d'ECG individuelle (30) par le dispositif de contrôle de qualité d'ECG (50) comprend en outre:
le dispositif de contrôle de qualité d'ECG (50) qui est adapté, pendant chaque segment d'électrocardiogramme, pour mettre à l'échelle tout changement du niveau de base de la dérivation d'ECG individuelle (30) par un facteur de pondération afin de dériver ainsi le score de bruit à basse fréquence de la dérivation d'ECG individuelle (30).

7. Dispositif de surveillance selon la revendication 5, dans lequel l'évaluation du niveau de bruit à basse fréquence de la dérivation d'ECG individuelle (30) par le dispositif de contrôle de qualité d'ECG (50) comprend en outre:
le dispositif de contrôle de qualité d'ECG (50) qui est adapté pour vérifier tout changement dans le niveau de base de la dérivation d'ECG individuelle (30) mesuré à un niveau d'activité spécifié de chaque segment d'électrocardiogramme.

8. Dispositif de surveillance selon la revendication 5, dans lequel l'évaluation du niveau de bruit à basse fréquence de la dérivation d'ECG individuelle (30) par le dispositif de contrôle de qualité d'ECG (50) comprend en outre:
le dispositif de contrôle de qualité d'ECG (50) qui est adapté pour comparer le score de bruit à basse fréquence avec au moins un seuil de qualité de signal différenciant une pluralité de zones de qualité de signal distinctes.

9. Dispositif de surveillance selon la revendication 1, dans lequel l'affichage de l'évaluation du niveau de bruit à haute fréquence et de l'évaluation du niveau de bruit à basse fréquence de la dérivation d'ECG individuelle (30) par le dispositif de contrôle de qualité d'ECG (50) comprend:

le dispositif de contrôle de qualité d'ECG (50) qui est adapté pour afficher l'un entre des dérivations d'ECG individuelles (30) ou un battement représentatif de la dérivation d'ECG (30) en même temps qu'une indication de qualité de signal des évaluations séparées du niveau de bruit à haute fréquence et du niveau de bruit à basse fréquence de la dérivation d'ECG individuelle (30),
où chaque indication de qualité de signal est au moins une indication de qualité de signal textuelle et/ou une indication de qualité de signal graphique.

10. Dispositif de surveillance selon la revendication 1, dans lequel le dispositif de contrôle de qualité d'ECG (50) comprend:

un estimateur de bruit à haute fréquence adapté pour calculer une médiane des écarts types de la dérivation d'ECG individuelle (30) mesurée à des niveaux d'activité spécifiés pendant un segment d'électrocardiogramme; et
un dispositif de notation de bruit à haute fréquence adapté pour dériver un score de bruit à haute fréquence à partir de un calcul par l'estimateur de bruit à haute fréquence de la médiane des écarts types pour la dérivation d'ECG individuelle (30); et
un comparateur de bruit à haute fréquence adapté pour comparer un calcul par le dispositif de notation de bruit à haute fréquence du score de bruit à haute fréquence avec au moins un seuil de qualité de signal différenciant une pluralité de zones de qualité de signal distinctes.

11. Dispositif de surveillance selon la revendication 10, dans lequel le dispositif de contrôle de qualité d'ECG (50) comprend en outre:

un indicateur de bruit à haute fréquence adapté pour dériver une indication de qualité de signal de l'évaluation du niveau de bruit à haute fréquence de la dérivation d'ECG individuelle (30) à partir d'une comparaison par le comparateur de bruit à haute fréquence du score de bruit à haute fréquence avec au moins un seuil de qualité de signal,
où chaque indication de qualité de signal est au moins une indication de qualité de signal textuelle et/ou une indication de qualité de signal graphique.

12. Dispositif de surveillance selon la revendication 1, dans lequel le dispositif de contrôle de qualité d'ECG (50) comprend:

un estimateur de bruit à basse fréquence adapté pour calculer tout changement dans un niveau de base de la dérivation d'ECG individuelle (30) à un niveau d'activité spécifié dans un segment d'électrocardiogramme; et
un dispositif de notation de bruit à basse fréquence adapté pour dériver un score de bruit à basse fréquence à partir d'un calcul par l'estimateur de bruit à basse fréquence de tout changement dans le niveau de base de la dérivation d'ECG individuelle (30); et
un comparateur de bruit à basse fréquence adapté pour comparer un calcul par le dispositif de notation de bruit à basse fréquence du score de bruit à basse fréquence avec au moins un seuil de qualité de signal différenciant une pluralité de zones de qualité de signal distinctes.

13. Dispositif de surveillance selon la revendication 12, dans lequel le dispositif de contrôle de qualité d'ECG (50) comprend en outre:

un indicateur de bruit à basse fréquence adapté pour dériver une indication de qualité de signal de l'évaluation du niveau de bruit à basse fréquence de la dérivation d'ECG individuelle (30) à partir d'une comparaison par le comparateur de bruit à basse fréquence du score de bruit à basse fréquence avec au moins un seuil de qualité de signal,
où chaque indication de qualité de signal est au moins une indication de qualité de signal textuelle et/ou une indication de qualité de signal graphique.

14. Procédé de surveillance, consistant à

dériver, par un électrocardiographe (40), un électrocardiogramme à partir d'au moins une dérivation d'ECG (30) en réponse à une connexion de l'électrocardiographe (40) à l'au moins une dérivation d'ECG (30);
contrôler, par un dispositif de contrôle de qualité d'ECG (50), des évaluations séparées d'un niveau de bruit à haute fréquence et d'un niveau de bruit à basse fréquence d'une dérivation d'ECG individuelle (30) sur la base d'une segmentation d'électrocardiogramme;
déterminer, lors de l'évaluation du niveau de bruit à haute fréquence de la dérivation d'ECG individuelle (30) par le dispositif de contrôle de qualité d'ECG (50), l'écart type d'un segment d'électrocardiogramme pour chaque dérivation d'ECG individuelle (30) par section de segment en tant qu'estimation du niveau de bruit à haute fréquence pour chaque dérivation d'ECG individuelle (30), le segment d'électrocardiogramme étant divisé en une pluralité de sections de segment; et
afficher, sur un écran de moniteur, les évaluations séparées du niveau de bruit à haute fréquence et du niveau de bruit à basse fréquence de la dérivation d'ECG individuelle (30) sur la base d'une segmentation d'électrocardiogramme.

ELECTROCARDIGRAM QUALITY CONTROLLER
50

| HIGH-FREQUENCY NOISE EVALUATION 51a (FIG. 2) | LOW-FREQUENCY NOISE EVALUATION 51b (FIG. 2) |
|---|---|

I  aVR  V1  V4
II  aVL  V2  V5
III  aVF  V3  V6

ELECTROCARDIOGRAPH
40

| I | aVR | V1 | V4 |
| II | aVL | V2 | V5 |
| III | aVF | V3 | V6 |

RA  LA
V1
V2-V6
RL  LL

FIG. 1A

FIG. 1B

FLOWCHART 70

| ACTIVITY MONITOR 51 | ⇨ | S72: ACTIVITY LEVEL COMPUTATION |

| HFN ESTIMATOR 52a | ⇨ | S74: NOISE ESTIMATION | ⇦ | LFN ESTIMATOR 52b |

| HFN SCORER 53a | ⇨ | S76: SCORE COMPUTATION | ⇦ | LFN SCORER 53b |

| HFN COMPARATOR 54a | ⇨ | S78: QUALITY DETERMINATION | ⇦ | LFN COMPARATOR 54b |

| HFN INDICATOR 55a | ⇨ | S80: QUALITY INDICATION | ⇦ | LFN INDICATOR 55b |

RETURN S72/TERMINATE

FIG. 2

FIG. 3

FIG. 4

UPPER LIMIT

'POOR'
SIGNAL QUALITY
ZONE

RED

THRESHOLD2

'FAIR'
SIGNAL QUALITY
ZONE

YELLOW

THRESHOLD1

'GOOD'
SIGNAL QUALITY
ZONE

GREEN

0

FIG. 5

FIG. 6

EP 3 340 871 B1

EP 3 340 871 B1

| H5 L6 | H5 L6 | H3 L2 | H2 L2 |
|-------|-------|-------|-------|
| H5 L6 | H4 L4 | H3 L3 | H3 L1 |
| H5 L2 | H5 L3 | H2 L2 | H3 L1 |

I                         aVR                        V1                       V4

II                        aVL                       V2                       V5

III                     aVF                       V3                       V6

II

## FIG. 7

## FIG. 8

EP 3 340 871 B1

| H5 L6 | H5 L6 | H3 L2 | H2 L2 |
|-------|-------|-------|-------|
| H5 L6 | H4 L4 | H3 L3 | H3 L1 |
| H5 L2 | H5 L3 | H2 L2 | H3 L1 |

I   aVR   V1   V4

II   aVL   V2   V5

III   aVF   V3   V6

FIG. 9

**EP 3 340 871 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100022903 A1
- US 5827196 A
- US 20140249437 A1